Europäisches Patentamt

(19) European Patent Office

Office européen des brevets

(11) EP 0 587 334 B1

(12) **EUROPEAN PATENT SPECIFICATION**

(45) Date of publication and mention
of the grant of the patent:
**30.06.1999 Bulletin 1999/26**

(51) Int. Cl.$^6$: **A61B 6/03**, A61B 6/00

(21) Application number: **93306595.5**

(22) Date of filing: **20.08.1993**

(54) **Imaging methods and apparatus**

Verfahren und Vorrichtung zur Bilderzeugung

Procédé d'imagerie et appareil associé

(84) Designated Contracting States:
**DE FR NL**

(30) Priority: **09.09.1992 US 943411**

(43) Date of publication of application:
**16.03.1994 Bulletin 1994/11**

(73) Proprietor:
**PICKER INTERNATIONAL, INC.**
**Highland Heights Ohio 44143 (US)**

(72) Inventor: **Heuscher, Dominic J.**
**Aurora, OH (US)**

(74) Representative:
**McGowan, Nigel George et al**
**The General Electric Company plc**
**GEC Patent Department**
**Waterhouse Lane**
**Chelmsford, Essex CM1 2QX (GB)**

(56) References cited:
**EP-A- 0 257 922     EP-A- 0 370 636**
**EP-A- 0 471 455     EP-A- 0 487 245**

## Description

This invention relates to imaging methods and apparatus.

More particularly the invention relates to the art of medical diagnostic imaging. It finds particular application in conjunction with spiral volume imaging with CT scanners and will be described with particular reference thereto. However, it is to be appreciated that the invention will also find application in conjunction with other types of volume imaging, with multiple single slice images, continuous rotating x-ray source images, gated imaging, and the like.

In spiral or helical scanning, the x-ray source or tube rotates continuously as the patient support table moves at a constant, linear velocity. In this manner, the collected data effectively represents a helical path of constant pitch through the patient. Conventionally, the data is stored and handled as a series of parallel planes, transverse to the longitudinal axis of the patient, or more specifically, as a three dimensional rectangular matrix of memory cells. See, for example, U.S. Patent No. 3,432,657 to Slavin.

For some medical diagnoses, it is advantages to inject a patient with a contrast agent, usually a high-Z contrast material such as iodine. Depending on how far the imaged region is from the heart, the contrast material maintains its peak concentration level in the region of interest over a period of about 30-90 seconds. CT scanning techniques have been developed for generating images of the region of interest while the contrast agent is near its peak. For example, ultra fast CT, i.e. electron beam scanning, has been used to cover a significant volume. See "Power-Injected CT Contrast Opacifies Vascular Spaces", Sam D. Lane, Diagnostic Imaging, November 1988. However, this technique cannot show contrast variations over a significant volume over a period of time. Lane must leave the couch in one position in order to show contrast variations of the same region over time. Thus, the Lane technique is disadvantageous due to the increased dose of contrast agent and the limiting of measurements to only a single observed axial slice.

A volume helical scanning technique is discussed in U.S. Patent No. 4,789,929 of Nishimura, et al. which utilizes back and forth motion of the couch to achieve more complete sampling of the volume being scanned. Nishimura does not address the concept in a temporal dimension. Moreover, the back and forth movement during data collection results in a temporally non-uniform sampling, particularly for regions of interest near the ends of the volume.

Spiral volume scans have been performed over a single patient breath-hold in the presence of a contrast agent. See "Spiral Volumetric CT with Single-Breath-Hold Technique, Continuous Transport, and Continuous Scanner Rotation", Radiology, Vol. 176, pp. 181-183, 1990 and "Physical Performance Characteristics of Spiral CT Scanning", Med. Phys. Vol. 18, No. 5, Sept./Oct. 1991, both by Kalender, et al. One drawback of these techniques is that they use a linear interpolator for the helical interpolation, which reduces sharpness of the CT slice definition. Another drawback is that only one set of measurements is obtained from a single breath-hold.

In order to fit the spiral collected data into a conventional three dimensional rectangular matrix, a series of parallel planes are defined through the spiral collected data, with a one plane per spiral revolution, e.g. at each 0° of source rotation. During the data collection period, a series of views or fans of data are collected at preselected angular increments around the patient. Potentially, one view per plane, by convention the 0° or 12 o'clock view, falls squarely in the plane requiring no averaging or weighting. For each remaining view of the plane, there is a pair of corresponding views or data fans, one from the revolution preceding the plane and the other from the revolution following the plane. These views are averaged or weighted in accordance with their relative distance from the plane. In this manner, a full set of weighted views is created to perform a conventional 360° CT reconstruction algorithm. See U.S. Patent No. 4,630,202 issued December 1986 to Mori.

One of the problems with the linear interpolation technique is that it introduces errors in fourth generation scanners using source fan reconstruction. In a third generation scanner in which the x-ray source and an arc of detectors rotate together about the slice, each data fan or view is collected instantaneously in a plane parallel to the artificially defined transverse slices. In a fourth generation scanner, there is a parallel ring of stationary detectors surrounding the patient. With source fan reconstruction, each detector is sampled at monitored, time displaced intervals generating a view or fan of data as the source rotates behind the examination region. Because the patient moves longitudinally between the first and last data sampling of the view or data fan, the views are warped or canted along the spiral path. The linear interpolation scheme which assumes that the views lie parallel to the artificially defined planes introduces errors.

Another problem with the linear interpolation technique is that it is particularly sensitive to variations in the x-ray rotation speed, the velocity with which the patient is moved, and fluctuations in the output of the x-ray tube.

EP-A-0 471 455 discloses a method of imaging including the step of: moving a radiation source and a subject relative to each other so as to irradiate the subject along a generally spiral path; and characterised in that said method further includes the steps of collecting a plurality of views of image data, each view being identifiable by an angular position around the subject and by an axial position along a spiral; interpolating corresponding views collected over more than two revolutions along generally spiral paths; and reconstructing the interpolated views into a plurality of image representations corresponding to a plurality of parallel slices through the subject.

EP-A-0 257 922 discloses a system for dynamic scan at cyclic displacement comprising: scanning means collecting projection data from a patient by scanning a portion whose position varies cyclically over a period of time; detecting means producing signals corresponding to the cyclical movement of said patient over said period of time; memory means memorising said projection data from said scanner means, and said signals from said detecting means correlating said projection data and said cyclical movement signals, means for setting a phase section at a specific phase in said scanned section of said patient; and reconstruction means for reconstructing the section which reads the necessary projection data for at least one plane of said images including said data corresponding to said set specific phase and reconstruct, a plurality of synchronised images in the time axis direction.

EP-A-0 487 245 discloses an apparatus for acquiring tomographic projection data of an imaged object comprising: means for supporting and translating the imaged object concurrently along a translation axis; means for projecting a beam of x-rays from an x-ray generator through the imaged object and alternately sweeping the beam along the translation axis in a first direction with translation of the imaged object during a first period, and in a second direction along the translation axis but counter to the translation of the imaged object during a second period; means for receiving the beam from the x-ray generator with an x-ray detector array after it passes through the imaged object; and means for holding the x-ray generator and x-ray detector in opposition around the imaged body and concurrently rotating the same around a center of rotation and the imaged object, in a gantry plane substantially perpendicular to the translation axis.

EP-A-0 370 636 discloses an apparatus for creating images representing partition coefficient and blood flow rates in a region of interest of a patient, the apparatus comprising: an enhancement agent means for introducing an enhancement agent into a patient's blood; a means for providing an indication of the concentration of the enhancement agent in the blood; and an imaging means for generating a plurality of time displaced electronic image representations of at least the region of interest, each image representation including a plurality of pixel values, each pixel value being indicative of enhancement agent concentration in a corresponding subregion of interest, characterised in that said apparatus further comprises: an absorption curve segment projecting means for projecting at least three absorption curve segments indicative of enhancement agent absorption by the blood during early, middle, and later time periods; and a partition coefficient and blood flow rate means for determining partition coefficients and blood flow rates from the pixel values and the projected absorption curve segments for subregions of the region of interest corresponding to the pixel values.

It is an object of the present invention to provide imaging methods and apparatus wherein one or more of the above problems are alleviated.

According to a first aspect of the present invention there is provided a method of diagnostic imaging comprising: (a) moving a subject and a radiation source relative to each other such that a volume of interest of the subject is irradiated along a generally spiral path of a preselected axial length over a preselected duration; (b) collecting a plurality of views of data each identifiable by a corresponding position along the spiral path; (c) interpolating axially corresponding views with an interpolation function; (d) reconstructing the views into a three-dimensional image representation; (e) repeating steps (a) - (d) a plurality of times and (f) interpolating the plurality of three-dimensional image representations in the temporal direction to create a temporally and spatially uniform image representation.

According to a second aspect of the present invention there is provided a diagnostic imaging apparatus comprising: means for repeatedly moving a subject and a radiation source such that the subject is irradiated along a generally spiral path over a preselected axial length over a preselected duration; a detector means for detecting radiation that has traversed the subject and producing a plurality of views of electronic data each identifiable by a corresponding position along the spiral path; means for interpolating axially corresponding views with an interpolating function; and means for reconstructing the views into a plurality of three-dimensional image representations; characterised in that the apparatus further includes means for interpolating the plurality of three dimensional image representations in the temporal direction to create a temporally and spatially uniform image representation.

One advantage of the present invention is that it enables contrast measurements of a volume to be taken over time.

Another advantage of the present invention is that it maintains a high volume of interest resolution both spatially, due to a high resolution interpolation and temporally, due to a rapid retrace and same direction spiral scanning.

Another advantage of the present invention is that flow and perfusion measurements can be made.

Another advantage of the present invention is that multiple-breath-hold measurements can be made.

One imaging method and apparatus in accordance with the invention will now be described, by way of example, with reference to the accompanying drawings in which:-

Figures 1A and B are a diagrammatic illustration of the apparatus;

Figure 2 illustrates the geometry of the apparatus;

Figures 3A-3D are illustrative of different weighting functions for weighting among more than two helical rotations in use of the apparatus;

Figures 4A and 4B are illustrative of interpolation or weighting functions for 180° based reconstruction algorithms;

Figures 5A and 5B illustrate contrast agent concentration versus time curves for the liver and aorta, respectively;

Figure 6 illustrates helical rotation angle, couch position, and couch velocity for a 15 cm volume covered using a 10 second scan at 1 second/revolution;

Figures 7A-7D are illustrative of compensations for gantry rotation and table speed variations, particularly variations in weighting functions for effecting the correction;

Figure 8 is analogous to Figure 6 but covering half the volume;

Figure 9 is analogous to Figure 6 for a 2.5 cm volume using a high resolution target scan;

Figure 10 is analogous to Figure 6 but provides time for breathing between scans;

Figures 11A-11C are illustrative of x-ray current and kV fluctuations and corresponding weighting functions for dual kV imaging; and,

Figure 12 illustrates a segmented detector array which enables multiple fan beams of data to be collected concurrently.

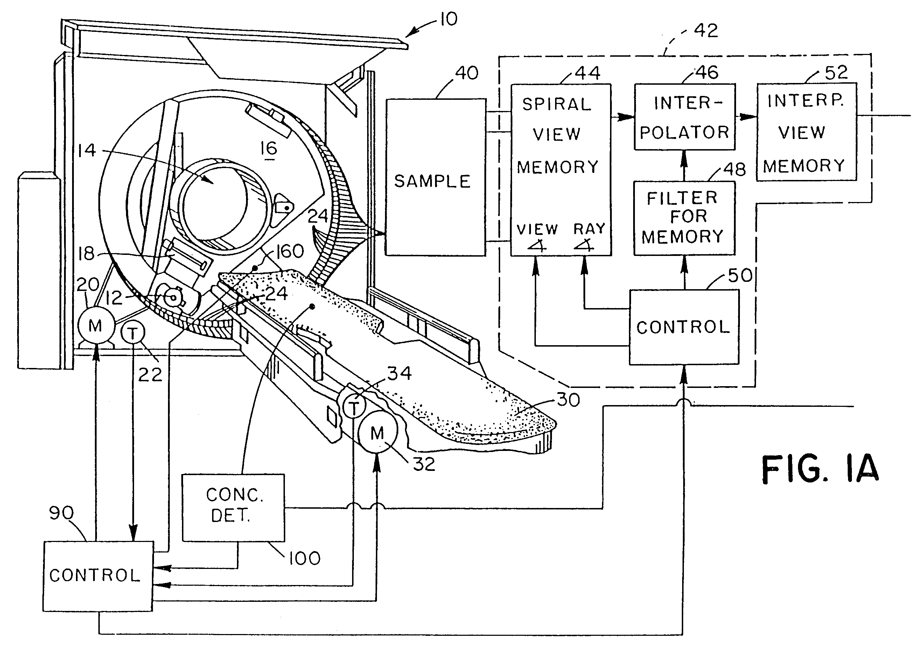

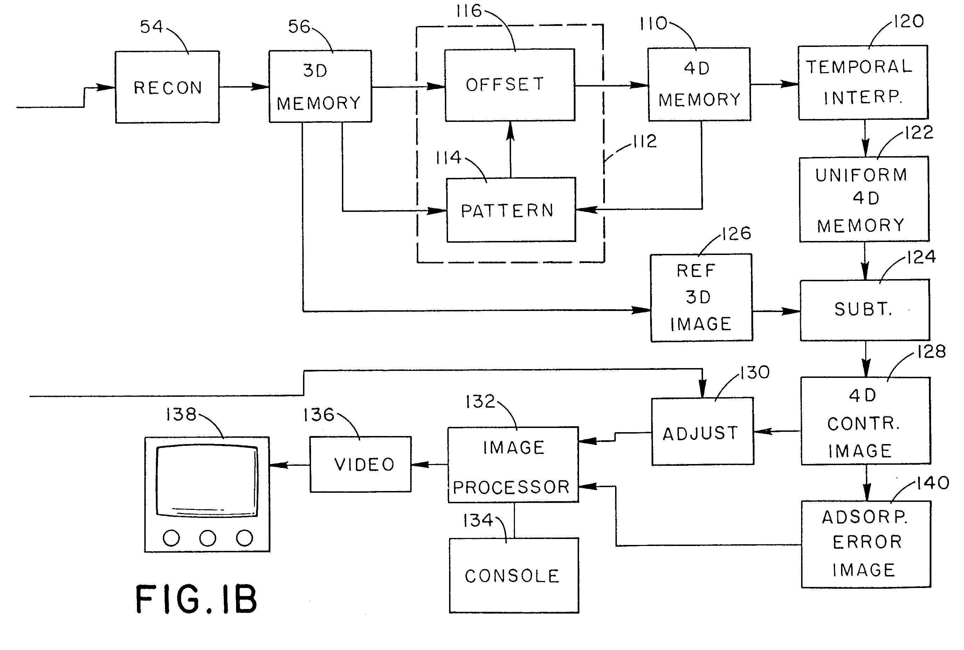

With reference to Figures 1A and 1B, the apparatus includes a CT scanner 10 including a radiation source 12, such as an x-ray tube, for projecting a fan beam of radiation through an examination region or scan circle 14. The x-ray tube is mounted on a rotatable gantry 16 to rotate the fan beam of radiation around the examination region. A collimator and shutter means 18 collimates the beam of radiation to one or more narrow planar beams and selectively gates the beam on and off. The beam may be also gated on and off electronically at the x-ray tube. A motor 20 provides motive power for rotating the gantry 16 continuously around the examination region. A rotational position encoder 22 is connected with the motor and the gantry to measure the rotational position of the gantry. In the illustrated fourth generation CT scanner, a ring of radiation detectors 24 are mounted peripherally around the examination region. For mechanical and mathematical convenience, the detectors 24 are stationarily mounted around the rotating gantry in the same plane as the x-ray tube.

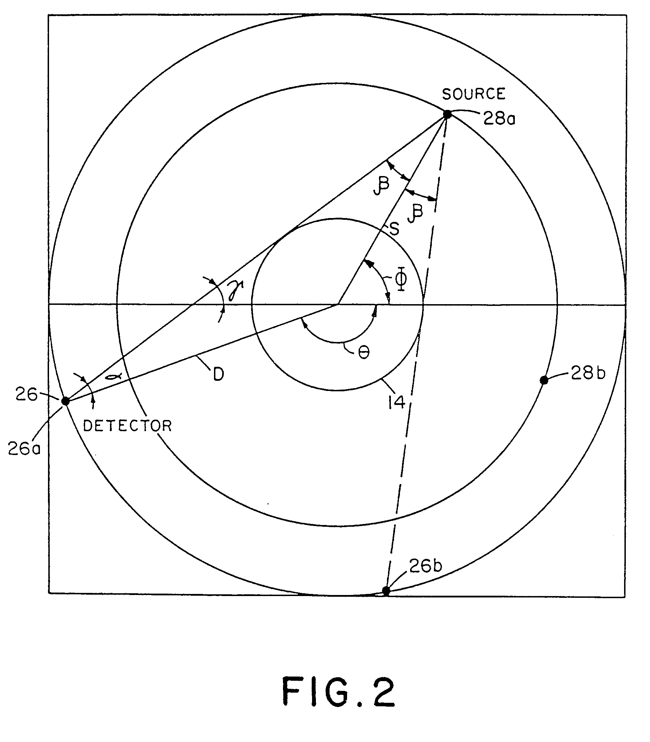

With reference to FIGURE 2, an arc of the detectors are sampled concurrently at short time increments as the radiation source 12 rotates in back of the examination region 14 to generate views or fan data sets. To generate detector fans which span an angle $2\alpha$, an exemplary detector 26 at an angular position $\theta$ is first sampled when the radiation source is at a location 28a tangent to one side of the examination region 14 and monitored incrementally until the radiation source reaches a point 28b in a line from the detector tangential to the other side of the examination region. For a source fan geometry, each of the detectors between detectors 26a and 26b are sampled concurrently to generate a source fan view or data set. The collected source fan data set can be identified by the angular position $\Phi$ of its apex around the examination region. Each ray of data between the source and one of the detectors is described by an angle $\beta$. Each ray of the source fan also is identifiable by its angle $\gamma$ relative to the common axis. The source is disposed a radius S from the center of the examination region and the ring of detectors 24 is disposed a radius D from the center of the examination region. In a third generation scanner in which the invention is equally applicable, a single arc of detectors between detectors 28a and 28b are mounted to the gantry 16 for rotation with the source. A third generation source fan geometry is described mathematically the same.

With reference again to FIGURE 1, a patient couch 30 supports a subject, particularly a human patient, in a reclined position. A means, such as a motor 32, advances the patient supporting surface of the couch through the examination region at a selectable velocity. An encoder 34 is connected with the motor 32, the moveable patient supporting portion 30, and the drive mechanism therebetween for monitoring the actual position of the patient supporting surface as it moves the patient through the scan circle 14.

A sampling means 40 samples the views or data sets corresponding to each angular position around the examination region 14 for each of a multiplicity of helical scans. A view processor 42 converts the spiral sampled views of each helical scan into a plurality of image representations corresponding to parallel planes sampled over a limited time range. The view processor includes a view memory 44 in which the view data stored and addressable by a combination of the scan number, (or time), rotation number, view angle, and ray angle within the view. The view processor 42 further includes a filter or interpolation means 46 for interpolating the data of each helical scan in the spiral view memory 44 into parallel slice data. The interpolation means 46 operates on a plurality of views of corresponding view angle with a filter or interpolation function supplied by a filter function memory 48.

A control means 50 indexes the view angle to each of the view angles in a complete helical scan, e.g. the views disposed at regular increments 360° around the examination region. A plurality of views corresponding to each individual view angle are transferred to the interpolation means to be filtered into an interpolated view. Each interpolated view is stored in an interpolated view memory means 52 until the interpolated views corresponding to each interpolated slice of one helical scan are generated. Thereafter, an image reconstruction means 54 uses a conventional filtered backprojection or other reconstruction algorithm to reconstruct each of a plurality of slices and store the resultant slices from each helical scan in a three-dimensional volume data memory 56. For each helical scan, the volume data memory means stores a rectangular pixel array corresponding to each of a plurality of slices, the slices being spaced a distance commensurate with the resolution of the pixels in each slice. In this manner, each set of data stored in the volume data memory means can be conceptualized as a rectangular data volume corresponding to one of the helical scans.

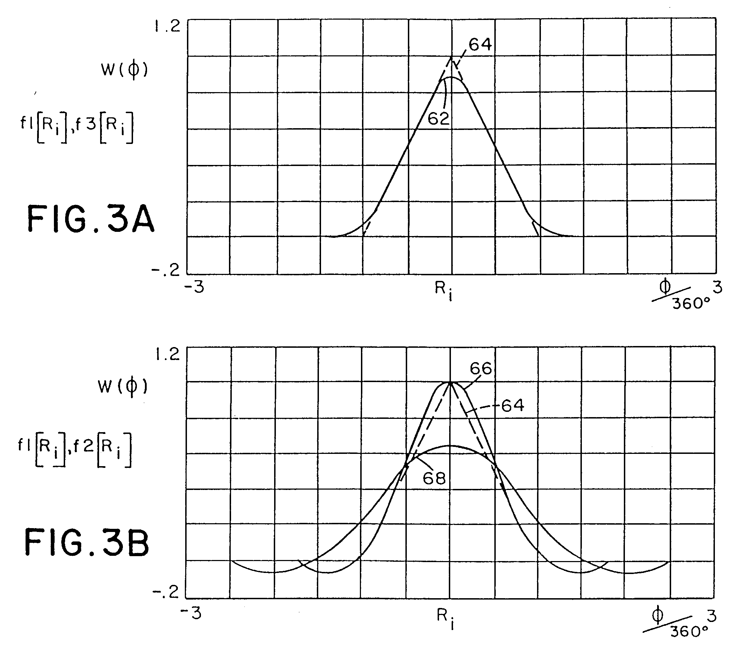

With reference to FIGURE 3A, the filter function memory means **48** stores a plurality of filter or interpolation functions. FIGURE 3A compares a modified linear weighting function **62** with a prior art linear weighting **64**. The modified linear weighting function $W(\phi)=0$ for $|R|>1.5$ with an average width of 1.1, where $\phi=R\cdot 360°$. $W(\phi)$ and its first derivative are both continuous as is represented by the rounding adjacent R=0 and R=±1. The rotational index R is the sum of the integral rotation index m and the fractional rotation r. Moreover, the weighting function has unity weighting, i.e.

$$\sum_{m} W(\phi_m)=1, \text{for } 0 <l<1, \tag{1}$$

where

$$\phi_m=(r+m)360°$$

and the first moments are equal to zero, i.e.

$$\sum_{m} (r+m)\cdot W(\phi_m)=0, \text{for } 0 <r<1. \tag{2}$$

In FIGURE 3B, a cubic weighting function **66** which spans four contiguous rotations is compared with the conventional linear interpolation **64**. In the cubic interpolation,

$$W(\phi)=0, \text{for}|R|>2 \tag{3}$$

with an average width $\approx 1.1$. $W(\phi)$ and its first derivative are again continuous and the conditions of Equations (1) and (2) are also valid. Analogously, a cubic weighting of the form **68** may also be utilized where:

$$W(\phi)=0, \text{for } |R|>3 \tag{4}$$

and with a width (at half height) equal to 1.6 rotations.

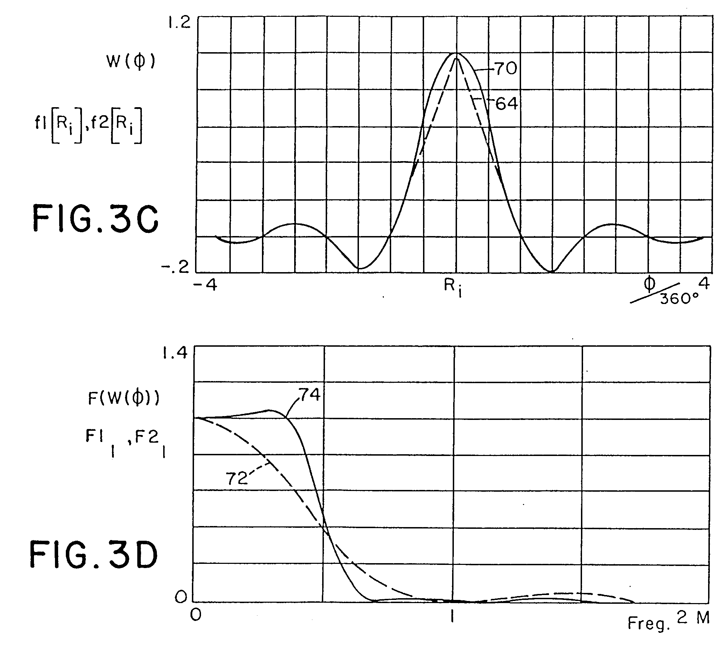

FIGURE 3C compares the conventional linear weighting **64** with a 7-lobe helical weighting function **70** where:

$$W(\phi)=0, \text{for } |R|>4 \tag{5}$$

Again, the function and its first derivative are continuous and the conditions of Equations (1) and (2) are met.

With reference to FIGURE 3D, the conventional linear weighting of curve **64** has a relatively limited frequency response **72** along the z-axis. By distinction, the 7-lobed helical weighting function **70** has a much crisper frequency response **74** in the z direction. Note that the frequency response of the 7-lobed helical weighting **74** is relatively flat and drops off relatively quickly which eliminates the smoothing heretofore required with the linear weighting function.

It should be noted that the revolution number or longitudinal position R in FIGURES 3A-3D is not measured digitally. That is, if the detector crosses the even integer values R=0, ±1, ±2, etc., at 0°, then at 90°, the R position is -.75, +.25, +1.25, etc.

In accordance with another embodiment of the present invention, the reconstruction means **54** uses a reconstruction algorithm based on views spanning 180° plus the fan angle, such as the algorithm illustrated in U.S. Patent No. 4,293,912 issued October 1981 to Walters. To utilize the 180° redundancy in the data most effectively with interpolation functions of the shapes described above but of half the extent, the interpolating filter from filter memory **48** is redefined on a 180° basis. Specifically, the linear weighting **64** would result in a warped rather than a planar image representation.

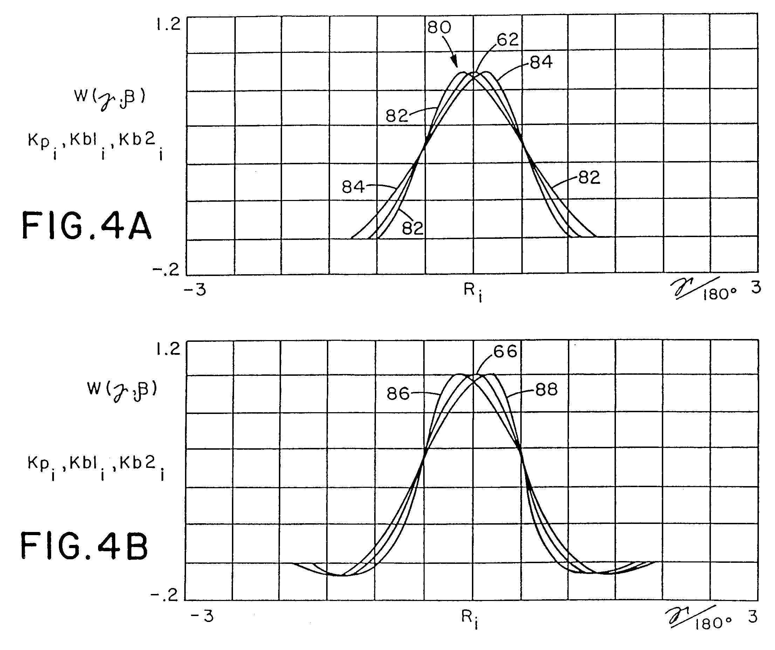

With reference to FIGURE 4A, a preferred technique for using 180° basis reconstruction techniques is to use a weighting function based on both the view angle and the angle of each individual ray within the view. Curve set **80** includes the modified linear function **62** for the center or zero ray of the view or fan. Curve **82** illustrates the modified weighting function shifted for the ray at one extreme of the fan and curve **84** illustrates the modified weighting function shifted for the ray at the opposite extreme of the fan. For each ray in between, the extreme and central rays, the weighting function is shifted a fraction of the illustrated difference. Each weighting curve and its first derivative are still continuous but now meet the conditions of Equations (6) and (7) below. The sum of the weighting functions for rays which are 180° opposite to each other is equal to one.

$$\sum_m W(\gamma_m, (-1)^m \beta)) = 1, \text{ for } \gamma_m = (r+m)180° \tag{6}$$

Also, the first moments are equal to zero, i.e.

$$\sum_m (r+m+\frac{(-1)^m \beta}{180}) \cdot W(\gamma_m, (-1)^m \beta) = 0. \tag{7}$$

For projection data organized in the form of source fans $V_S(\Phi+n \cdot 360°, \beta)$, the longitudinal interpolation generates:

$$D_{180}(\phi, \beta) = \sum_n W_S(\phi+n \cdot 360°, \beta) \cdot V_S(\phi+n \cdot 360°, \beta) \tag{8a}$$

where:

$$W_S(\phi+n \cdot 360°, \beta) = W(\gamma+\beta, \beta)$$

and $W_S(\gamma, \beta)$ is the weighting function that satisfies conditions (6) and (7) and $\phi = 0$ to 360°

$$\beta = \frac{-\beta_0}{2} \text{ to } \frac{\beta_0}{2}$$

$\beta_0$ = fan angle defining the scan circle.

The resulting 360° of projection data $D_{180}(\phi, \beta)$ no longer has unity weighting, but instead has an average weighting spanning 180°. This data can be reconstructed using standard convolution backprojection of $D_{180}(\phi, \beta)$ for $\phi$ = 0 to 360°. For projection data organized as detector fans $V_D(\theta+n \cdot 360°, \alpha)$, the longitudinal interpolation generates:

$$D_{180}(\theta, \alpha) = \sum_n W_D(\theta+n \cdot 360°, \alpha) \cdot V_D(\theta+n \cdot 360°, \alpha) \tag{8b}$$

where $W_D(\theta+n \cdot 360°, \alpha)$ is obtained by remapping $W_S(\phi+n \cdot 360°, \beta)$ from source fan format to detector fan format. Again, this data can be reconstructed using standard convolution backprojection of $D_{180}(\theta, \alpha)$ for $\theta = 0$ to 360°.

Analogously, the weighting function $W_{180}(\gamma, \beta)$ for the cubic weighting function of curve **66** of FIGURE 3B can be made ray dependent as illustrated in FIGURE 4B. Specifically, cubic weighting function **66** is used for the central ray. For the ray at one extreme, the weighting function is distorted as illustrated by curve **86** and for the ray of the other extreme of the fan, the weighting curve is distorted as illustrated at **88**. In the illustrated embodiment, the extreme rays of curves **86** and **88** are for $\beta = \pm 23°$.

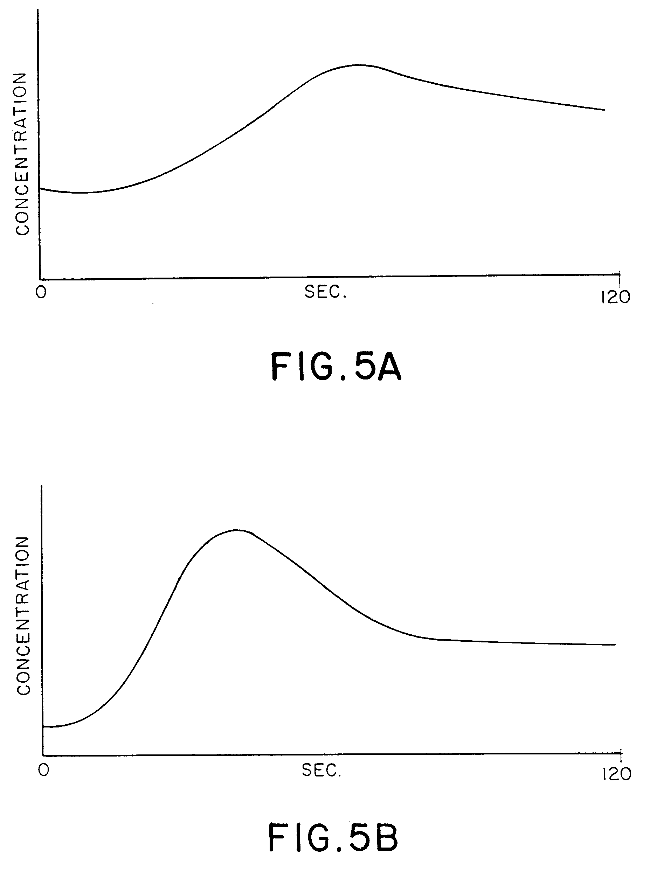

In contrast imaging, a contrast agent is injected into the patient. The concentration of contrast agent at a region of interest varies with time in accordance with a distance of the region of interest from the heart, the rate of injection, and the like. FIGURE 5A is typical of contrast agent concentration versus time for the liver and FIGURE 5B is typical of contrast agent versus time for the aorta. For four-dimensional images, the above-described imaging process is repeated a plurality of times while the concentration of the contrast agent is near its peak. Additionally, three-dimensional volumetric data sets collected before and after the peak in order to provide a baseline for the contrast agent.

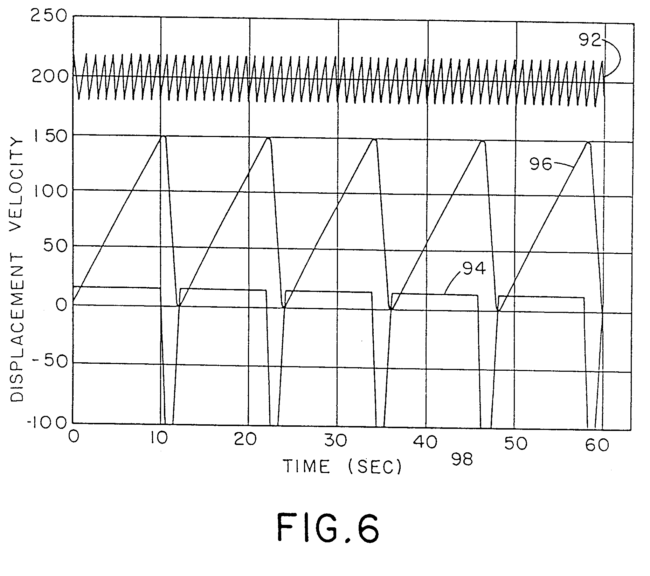

With reference again to FIGURE 1 and further reference to FIGURE 6, a control means **90** controls the motors **20** and **32** such that the x-ray source is rotated **92** at about 1 second/revolution and the patient table is moved **94** at about 15 mm/second. This enables a volume which is 15 cm in the axial direction **96** to be covered in 10 seconds. The control **90** then reverses the couch control motor **32** and operates it a higher speed, e.g. 7.5 cm/second, such that the couch is returned **98** to its starting position in about 2 seconds. The control **90** also gates the x-ray tube off or closes the shutter **18** such that the patient is not irradiated during the return or retrace period. This process is then repeated, generating a full set of volume image data about every 12 seconds, i.e. 5 sets/minute.

A concentration agent concentration means **100** signals the control means **90** when the scans are to begin. The contrast agent concentration means 100 may monitor the actual concentration of the contrast agent within the patient. Preferably, because the time from the introduction of the contrast agent until it approaches its peak concentration is well-established medically, the contrast agent concentration means simply times the appropriate duration between introduction of the contrast agent until the known time parameter is reached.

The speed of the table is maintained substantially constant. However, any acceleration at the beginning and deceleration at the end of each helical scan can be readily handled during the interpolation. Moreover, there can be variations in the speed along the scan due to wear, power fluctuations, and the like.

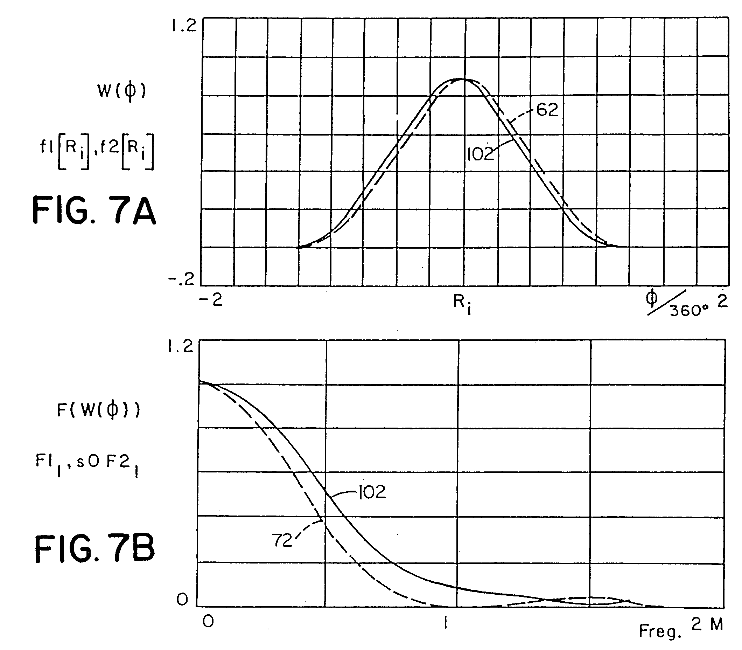

With reference to FIGURE 7A, interpolation function **62** is recalculated to function **102** to account for a $\pm$ 20% sinusoidal speed variation. Specifically:

$$\sum_m W_A(\phi_m)=1, \text{ for } \phi_m=(r+m) \cdot 360 \tag{9}$$

$$\sum_m P(\phi_m) \cdot W_A(\phi_m)=0, \tag{10}$$

where $P(\phi)$ is the table position relative to rotational angle $\phi$. Although this satisfies the requirement for planar reconstructions, FIGURE 7B shows that the spatial resolution of curve **102** does not match the ideal linear weighting function response **72**. However, if the values of the weighting function $W(\phi)$ are remapped to $W(P(\phi))$, a constant spatial response can be maintained. This weighting is adjusted such that:

$$\sum_m W_A(P(\phi_m))=1, \text{ for } \phi_m=(r+m) \cdot 360° \tag{11}$$

$$\sum_m P(\phi_m)W_A(P(\phi_m))=0. \tag{12}$$

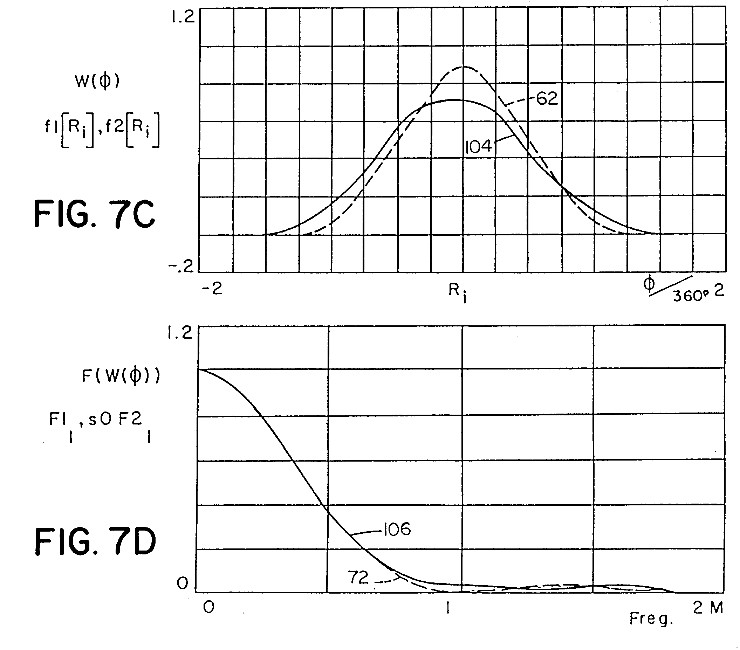

With the new weighting function **104** of FIGURE 7C, a spatial response **106** of FIGURE 7D is substantially the same as the spatial response **72** of FIGURE 7B. In this manner, the interpolation function is utilized to correct for fluctuations in the speeds of relative movement of the source and patient.

As each repetition of the spiral data collection is commenced, the three-dimensional reconstructed image representation is moved from the three-dimensional image memory **56** to a four-dimensional image memory **110**, where the fourth dimension is time. An alignment means **112** spatially aligns each three-dimensional volume image with the preceding volume images. A pattern recognition means **114** examines each three-dimensional image representation for one or more characteristic points or structures and compares the spatial location of these characteristic points or structures with the location of the same characteristic point in the four-dimensional memory **110**. The characteristic points may be defined by placing x-ray opaque alignment members on the surface of the patient, whose coordinate location in each volume image representation is compared with its location in the preceding volume image representations. An offset means **116** adds an appropriate spatial offset to each image representation in order to bring the characteristic point of the newly acquired volume image representation into alignment with the corresponding characteristic points of previously collected volume image representations. Optionally, the means **116** may also scale the 3D image representation to match the previously acquired data more precisely. Alternately, the recognition means **114** may include a pattern recognition means which examines each image for a characteristic anatomical pattern and compares the position of this characteristic anatomical pattern with the position of the same anatomical pattern in the previously acquired volume image representations in the four-dimensional memory means **110**.

As discussed above, each of the spiral data acquisition periods takes some time, 10 seconds in the above example. This causes the data within each three-dimensional image representation to be skewed in time. More specifically, in the above-discussed embodiment, the data at the first end of the volume leads the data at the second end by about 10 seconds. The data at the first end of the next volume trails the data at the second end of the preceding data set by about 2 seconds and the data at the beginning of the last data set by about 12 seconds. A temporal interpolating means **120** interpolates the four-dimensional data in the temporal direction to create data sets which are at even temporal intervals. The temporal and spatially uniform four-dimensional data is stored in a four-dimensional uniform image memory means **122**.

For diagnostic purposes, it is often advantageous to generate a display of contrast agent alone. To this end, a subtraction means **124** subtracts a three-dimensional basis or zero contrast agent three-dimensional image from each of the temporally uniform three-dimensional volume images in the uniform volume image memory means **122**. More specifically, the control means **90** causes a three-dimensional image taken before the contrast agent is introduced to be loaded into a three-dimensional reference image memory means **126**. Optionally, the control means may cause another image to be taken after the contrast agent should have passed out of the region of interest. These before and after three-dimensional image representations can be averaged to form the basis image for storage in the basis volume image memory means **126**. Once the substraction means **124** subtracts the basis volume image from each of the temporally displaced volume images, the remainder is a four-dimensional image of the elevation of the contrast agent as a function of time, which contrast agent image is stored in a four-dimensional contrast agent image memory means **128**.

As indicated in FIGURES 5A and 5B, the amount of contrast agent within the imaged volume varies with a curve. Where appropriate, an adjustment to the concentration image can be made to account for this temporal variation in concentration agent dose. To this end, a concentration image adjusting means **130** adjusts the four-dimensional contrast image, as may be desired, to account for the temporal variation in contrast image dose. The contrast image concentration curve can be supplied by the contrast agent detection means **100**, either by actually measuring the contrast agent or with a priori information. Further, the contrast agent curve can be derived from the contrast agent image **128**.

An image and video processor means **132** is controlled by a console **134** to select two-dimensional images from the four-dimensional image data for display. The processor means selects various two-dimensional image representations, such as planar slices through the volume at various angles and at various times, series of images through the same slice showing the evolution over time, projection images, segmented or cut away images, or the like. Moreover the processing means **132** can superimpose data from the reference image from memory **126** with the contrast image **128**. For example, it is often advantageous to see the surrounding tissue which is not affected by the contrast agent in order to assist in spatially orienting the radiologist. The background tissue may be displayed in black and white while the contrast image is displayed in other single or multiple color presentations superimposed thereon. A video processor **136** converts the selected two-dimensional image representation(s) into appropriate format for display on a video monitor **138**. Optionally, additional image processing means, image memory storage and archiving means, and the like may be provided.

The rate at which the contrast agent reaches each pixel of the image has diagnostic significance. Ideal contrast agent concentration curves for the liver and aorta are shown in FIGURES 5A and 5B. However, because the contrast agent is carried by the blood, failure of the contrast agent to reach a specific pixel in a comparable length of time is indicative of circulatory or other metabolic problems. Optionally, a means **140** examines the corresponding voxel of each of the time displaced three-dimensional images, plots the contrast agent concentration, and compares it to the ideal. From this information, a three-dimensional representation is generated indicating whether each voxel of the volume is receiving the concentration at the proper rate and if not, by how much it is slow. By displaying normal in one color or shade, and regions which receive the contrast agent progressively under the predicted rate in progressively varying colors or shades, valuable diagnostic information can be conveyed. The individual contrast agent versus time curve for each voxel may be displayed individually.

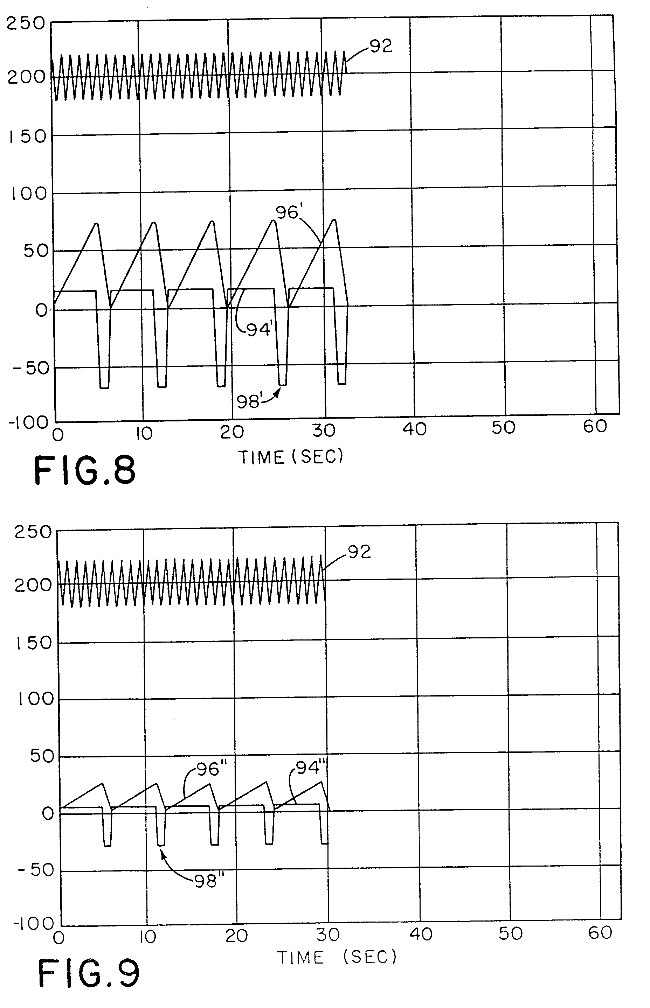

Other volume temporal measurement formats may be selected. With reference to FIGURE 8, the control means **90** can again rotate **92** the gantry at 1 second/revolution. In this embodiment however, the patient is moved **94'** axially only 7.5 cm/scan and the table retraces **98'** the 7.5 cm in about 1.5 seconds. Again, the couch velocity **96'** is substantially constant over the 5 second helical scanning time. In this embodiment, a volume half as large as in the embodiment of FIGURE 6 but is scanned every 6.5 seconds, enabling 8 full scans per minute to be achieved.

With reference to FIGURE 9, the gantry again rotates **92** at about 1 second/revolution. However, for higher resolution, the table moves **94"** only 2.5 cm in the period of about 5 seconds. This enables the table to be stopped and returned **98"** to the original position in about a second for about a 6 second scan. Thus, in this embodiment, 10 helical scans per minute can be achieved.

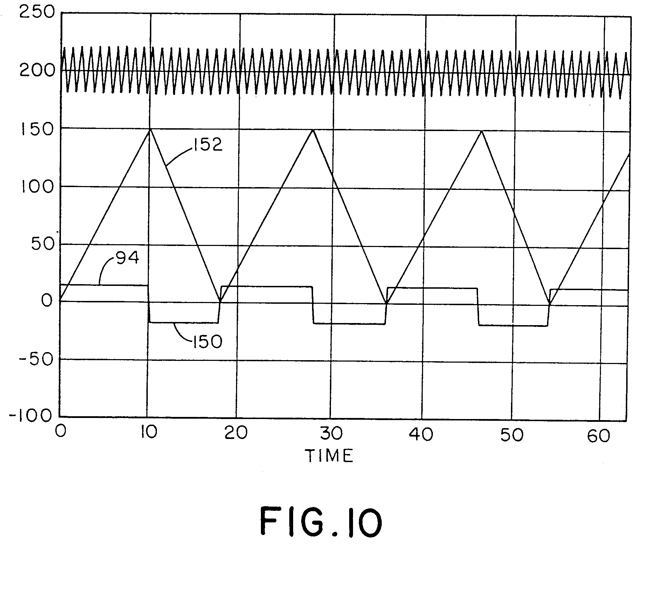

With reference to FIGURE 10, the patient table scans 15 cm in about 10 seconds **94** while the patient holds his breath. A period **150** of about 8 seconds is provided for the patient to exhale and inhale again in preparation for the next breath-hold. During which breathing time the table is returned **152** to its initial starting position. In this manner, a scan can be achieved about every 18 seconds or about 4 scans in 64 seconds.

A human patient commonly has an oval cross section which changes the dynamic range of transmitted radiation with angular orientation. The synchronization control means **90** further controls a power supply **160** for the x-ray tube **20**. The power supply adjusts the x-ray tube current or voltage generally in proportion to the mean attenuation path through the patient for each angular position of the x-ray tube. By varying the exposure in this manner, the quantum noise statistics of the reconstructed volume can be made more uniform.

When the gantry rotation or table speed are varied disproportionately, the scan becomes non-helical. However, (as demonstrated above), a suitable reconstruction volume can still be attained. For higher attenuation projections, either the gantry rotation or the table movement is slowed to compensate for the higher quantum noise. Analogously, if the x-ray tube voltage, the filtration, or the x-ray tube current is varied, a helical scan can still be performed. Specifically, the voltage filtration and/or current are increased for higher attenuative projections. The synchronization of these parameters may be either predetermined based on previous estimates or may be determined by estimates from earlier projections or scans of the same volume.

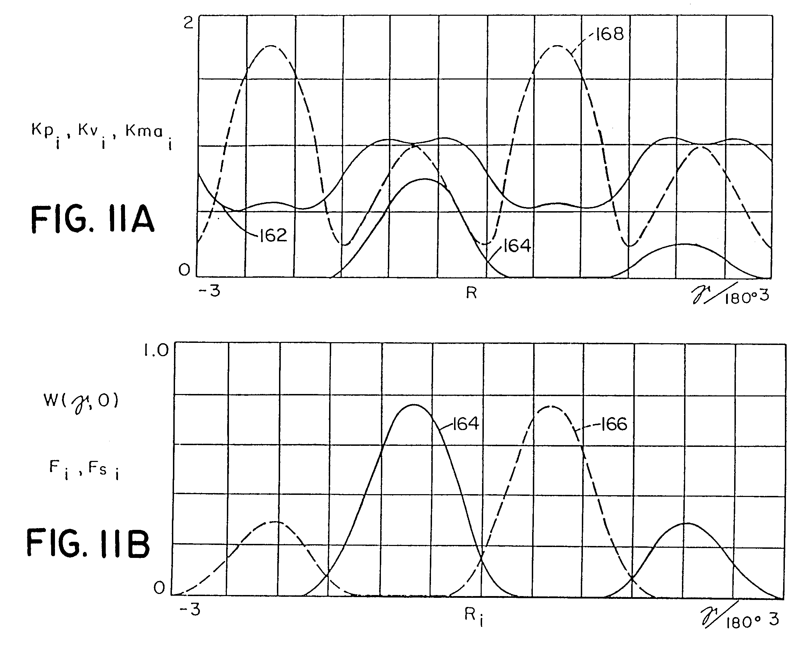

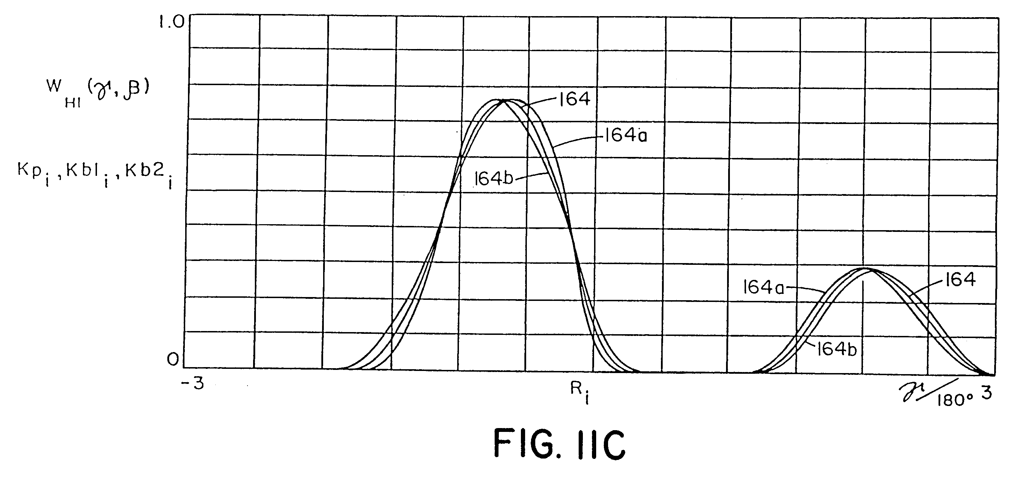

As discussed above, the views may be grouped into a group spanning 360° for 180° based reconstructions. The energy level (kV) of the x-ray tube in another embodiment is varied or alternated between two levels. By continuously varying the kV with a prescribed high to low variation as shown in **162** of FIGURE 11A, two weighting functions (**164, 166**) can be applied to the respective projections to produce two sets of 180° based projections, both of which correspond to the same imaging plane. The average kV value for each ray in each set corresponds to either the high kV level or low kV level. Exactly 1-1/2 rotations are required for each cycle from high kV to low kV back to high kV. The x-ray current **168** is varied counter cyclically to maintain the noise in both the high and low kV projections while minimizing the total exposure. Curve **164** identifies the high kV weighting function, $W_{hi} (R \cdot 180, \beta) = W_{hi} (\gamma, \beta)$.

With reference to FIGURE 11B, the weighting function applied by the interpolating means **46** shifts for the high and low kV portions. That is, the weighting function curve **164** illustrates the preferred weighting function for the high kV or voltage projection rays whereas the weighting function curve **166** illustrates the weighting function used with the low energy rays. These ray projections are recombined into two separate 1800 based sets or groups of projections. In the illustrated embodiment, the beam width corresponds to about three rings of the helix and a reconstructed image or slice is obtained for every one and a half rings of the helix.

With reference to FIGURE 11C, the variation in weighting values for the extremes of the fan ($\beta = \pm\beta_o$) is illustrated for the high kV 180° based projection set. More specifically, weighting function curve **164** is shifted or swayed between curve **164a** at one extreme ray of the fan and **164b** at the other extreme ray of the fan. Analogous shifts are made for the weighting function **166** for the low kV projection set.

To increase the x-ray collection efficiency, a plurality of detectors are positioned adjacent to each other in the longitudinal direction. Positioning two detectors longitudinally enables the width of the radiation seen by each detector to be selectively adjusted at the detector. Analogously, three or more detectors can be disposed in longitudinal alignment. This enables data along three interleaved spirals to be collected concurrently. In one embodiment, the three spirals of data cover the same volume with a greater sampling density. This is particularly advantageous in the dual energy modes described above. Alternately, the speed of the patient table is tripled such that the three sets of detector collect data with the same sampling density but three times as fast.

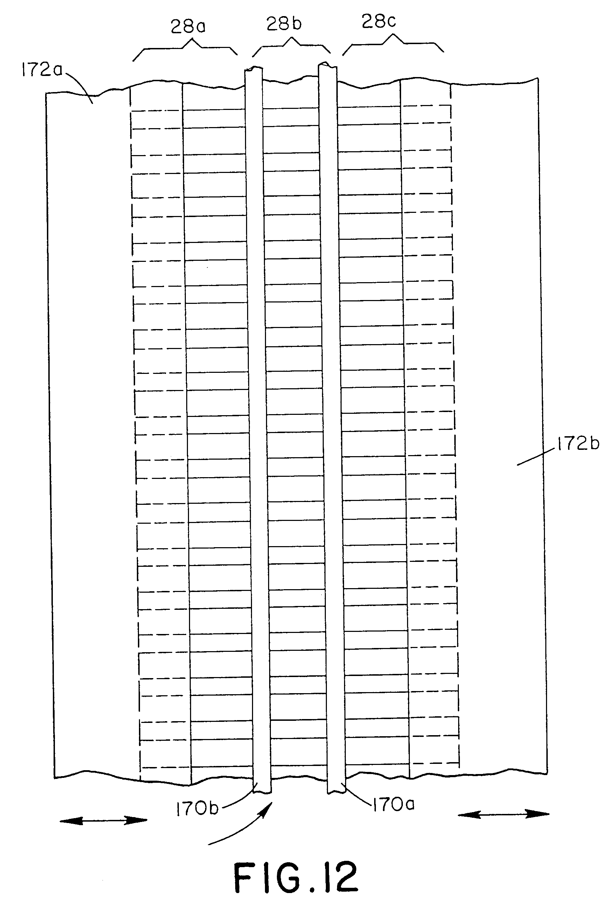

With reference to FIGURE 12, the detector array **24** preferably includes a plurality of rings of detectors **28a**, **28b**, **28c**. Collimators **170a**, **170b** selectively adjust the width of the beam striking the central detector ring **28b**. Outer collimators **172a**, **172b** adjust the widths of the x-ray beams striking the outer detector rings **28a**, **28c**. In this manner, three x-ray beams are generated to collect data corresponding to three helices concurrently. Alternatively, a multi-spot x-ray tube may be utilized to increase the thickness of the x-ray beam such that the plurality of beams are more parallel.

The invention has been described with reference to the preferred embodiment. Obviously, modifications and alterations will occur to others upon reading and understanding the preceding detailed description. It is intended that the invention be construed as including all such modifications and alterations insofar as they come within the scope of the appended claims, since the invention is defined by the appended claims.

## Claims

**1.** A method of diagnostic imaging comprising: (a) moving a subject and a radiation source relative to each other such that a volume of interest of the subject is irradiated along a generally spiral path of a preselected axial length over a preselected duration; (b) collecting a plurality of views of data each identifiable by a corresponding position along the spiral path; (c) interpolating axially corresponding views with an interpolation function; (d) reconstructing the views into a three-dimensional image representation; (e) repeating steps (a) - (d) a plurality of times and (f) interpolating the plurality of three-dimensional image representations in the temporal direction to create a temporally and spatially uniform image representation.

2. A method as claimed in Claim 1, wherein a three dimensional image representation of a volumetric region of the subject is subtracted from a temporally uniform image of the same region of the subject to generate an image representation representing change in the volume of interest with time.

3. A diagnostic imaging apparatus comprising: means (20, 32) for repeatedly moving a subject and a radiation source (12) such that the subject is irradiated along a generally spiral path over a preselected axial length over a preselected duration; a detector means (24) for detecting radiation that has traversed the subject and producing a plurality of views of electronic data each identifiable by a corresponding position along the spiral path; means (46) for interpolating axially corresponding views with an interpolating function; and means (54) for reconstructing the views into a plurality of three-dimensional image representations; characterised in that the apparatus further includes means (120) for interpolating the plurality of three dimensional image representations in the temporal direction to create a temporally and spatially uniform image representation.

4. An apparatus as claimed in Claim 3 further including means (124) for subtracting a three dimensional image representation of a volumetric region of the subject from a temporally uniform image of the same region of the subject to generate an image representation representing change in the volume of interest with time.

**Patentansprüche**

1. Ein Verfahren zur diagnostischen Bilderzeugung, das beinhaltet: (a) Bewegung eines Objekts und einer Strahlungsquelle relativ zueinander, so daß ein interessierendes Volumen des Objektes längs eines im allgemeinen spiralförmigen Pfades einer festgesetzten axialen Länge während einer festgesetzten Zeitspanne bestrahlt wird; (b) Aufnahme mehrerer Datenansichten, wovon jede durch eine entsprechende Position entlang des spiralförmigen Pfades identifizierbar ist; (c) Interpolation axial entsprechender Ansichten mit einer Interpolationsfunktion; (d) Rekonstruktion der Ansichten zu einer dreidimensionalen Bilddarstellung; (e) mehrmalige Wiederholung der Schritte (a)-(d); und (f) Interpolation der mehreren dreidimensionalen Bilddarstellungen in zeitlicher Richtung, um eine zeitlich und räumlich gleichförmige Bilddarstellung zu erhalten.

2. Ein Verfahren nach Anspruch 1, wobei eine dreidimensionale Bilddarstellung eines Volumenbereiches des Objektes von einem zeitlich gleichförmigen Bild desselben Bereiches des Objektes subtrahiert wird, um eine Bilddarstellung zu erzeugen, die Änderungen in dem interessierenden Volumen im Zeitablauf darstellt.

3. Eine diagnostische Vorrichtung zur Bilderzeugung, die aufweist: Einrichtungen (20, 32) zur wiederholten Bewegung eines Objektes und einer Strahlungsquelle (12), so daß das Objekt längs eines im allgemeinen spiralförmigen Pfades über eine festgesetzte axiale Länge während einer festgesetzten Zeitspanne bestrahlt wird; eine Detektoreinrichtung (24) zur Detektion der Strahlung, die das Objekt durchquert hat, und zur Erzeugung mehrerer Ansichten elektronischer Daten, wovon jede durch eine entsprechende Position entlang des spiralförmigen Pfades identifizierbar ist; Einrichtungen (46) zur Interpolation axial entsprechender Ansichten mit einer Interpolationsfunktion; und Einrichtungen (54) zur Rekonstruktion der Ansichten zu mehreren dreidimensionalen Bilddarstellungen; **dadurch gekennzeichnet**, daß die Vorrichtung weiterhin Einrichtungen (120) zur Interpolation der mehreren dreidimensionalen Bilddarstellungen in zeitlicher Richtung aufweist, um eine zeitlich und räumlich gleichförmige Bilddarstellung zu erzeugen.

4. Eine Vorrichtung nach Anspruch 3, die weiterhin Einrichtungen (124) zur Subtraktion einer dreidimensionalen Bilddarstellung eines Volumenbereiches des Objektes von einem zeitlich gleichförmigen Bild desselben Bereiches des Objektes aufweist, um eine Bilddarstellung zu erzeugen, die Änderungen in dem interessierenden Volumen im Zeitablauf darstellt.

**Revendications**

1. Procédé d'imagerie de diagnostic, comprenant (a) le déplacement d'un sujet et d'une source de rayonnement l'un par rapport à l'autre, afin qu'un volume intéressant du sujet soit irradié le long d'un trajet spiralé de façon générale de longueur axiale prédéterminée et pendant une durée prédéterminée, (b) la collecte de plusieurs vues de données qui peuvent être identifiées chacune par une position correspondante le long du trajet spiralé, (c) l'interpolation axiale de vues correspondantes par une fonction d'interpolation, (d) la reconstruction des vues sous forme d'une représentation d'images tridimensionnelles, (e) la répétition des étapes (a) à (d) plusieurs fois, et (f) l'interpolation des représentations tridimensionnelles d'image dans la direction temporelle pour la création d'une représentation d'image uniforme dans le temps et dans l'espace.

2. Procédé selon la revendication 1, dans lequel une représentation d'image tridimensionnelle d'une région volumétrique du sujet est soustraite d'une image uniforme dans le temps de la même région du sujet pour la création d'une représentation d'image qui représente un changement au cours du temps dans le volume intéressant.

3. Appareil d'imagerie de diagnostic, comprenant un dispositif (20, 32) de déplacement répété d'un sujet et d'une source de rayonnement (12) afin que le sujet soit irradié le long d'un trajet spiralé de façon générale sur une longueur axiale prédéterminée et pendant une durée prédéterminée, un dispositif détecteur (24) destiné à détecter le rayonnement qui a traversé le sujet et à produire plusieurs vues de données électroniques qui peuvent être identifiées chacune par une position correspondante le long du trajet spiralé, un dispositif (46) d'interpolation de vues qui se correspondant axialement avec une fonction d'interpolation, et un dispositif (54) de reconstruction des vues en plusieurs représentations d'images tridimensionnelles, caractérisé en ce que l'appareil comporte en outre un dispositif (120) d'interpolation des représentations d'images tridimensionnelles dans la direction temporelle pour la création d'une représentation d'image uniforme dans le temps et dans l'espace.

4. Appareil selon la revendication 3, comprenant en outre un dispositif (124) de soustraction d'une représentation d'image tridimensionnelle d'une région volumétrique du sujet d'une image uniforme dans le temps de la même région du sujet pour la création d'une représentation d'image qui représente un changement au cours du temps dans le volume intéressant.

FIG. IA

EP 0 587 334 B1

FIG. IB

# FIG.2

EP 0 587 334 B1

$W(\phi)$

$f1[R_i], f3[R_i]$

FIG.3A

1.2

64

62

-.2

-3

$R_i$

$\dfrac{\phi}{360°}$ 3

$W(\phi)$

$f1[R_i], f2[R_i]$

FIG.3B

1.2

66

64

68

-.2

-3

$R_i$

$\dfrac{\phi}{360°}$ 3

FIG.3C

FIG.3D

FIG.4A

$W(\gamma, \beta)$

$Kp_i, Kb1_i, Kb2_i$

80 62 84 82

84 82 82

$-3$   $R_i$   $\gamma/_{180°}$  3

FIG.4B

$W(\gamma, \beta)$

$Kp_i, Kb1_i, Kb2_i$

66 86 88

$-3$   $R_i$   $\gamma/_{180°}$  3

EP 0 587 334 B1

FIG.5A

FIG.5B

FIG.6

W(φ)

f1[Rᵢ],f2[Rᵢ]

## FIG. 7A

F(W(φ))

FI₁,sO F2₁

## FIG. 7B

EP 0 587 334 B1

FIG. 7C

$W(\phi)$

$f1\left[R_i\right], f2\left[R_i\right]$

FIG. 7D

$F(W(\phi))$

$F1_i, sO\ F2_i$

FIG.8

FIG.9

FIG.10

EP 0 587 334 B1

$Kp_i, Kv_i, Kma_i$

## FIG. IIA

168

162

164

2

0

−3

R

$\mathcal{Y}/180°$ 3

$W(\mathcal{Y}, 0)$

$F_i, Fs_i$

## FIG. IIB

164

166

1.0

0

−3

$R_i$

$\mathcal{Y}/180°$ 3

FIG. IIC

EP 0 587 334 B1

FIG.12